# EUROPÄISCHE PATENTSCHRIFT

(11) **EP 4 312 043 B1**
(45) Veröffentlichungstag und Bekanntmachung des Hinweises auf die Patenterteilung: **22.01.2025**
(21) Anmeldenummer: 22186742.7
(22) Anmeldetag: 25.07.2022
(51) Int. Cl.: G01R 33/54

(54) **MAGNETRESONANZTOMOGRAPH UND VERFAHREN FÜR EINEN ENERGIESPARENDEN BETRIEB DES MAGNETRESONANZTOMOGRAPHEN**
MAGNETIC RESONANCE TOMOGRAPH AND METHOD FOR ENERGY-SAVING OPERATION OF THE MAGNETIC RESONANCE TOMOGRAPH
TOMOGRAPHE À RÉSONANCE MAGNÉTIQUE ET PROCÉDÉ DE FONCTIONNEMENT DU TOMOGRAPHE À RÉSONANCE MAGNÉTIQUE DE MANIÈRE ÉCONOMIQUE EN ÉNERGIE

(43) Veröffentlichungstag der Anmeldung: 31.01.2024
(73) Patentinhaber: Siemens Healthineers AG, 91301 Forchheim (DE)
(72) Erfinder: Beck, Thomas, 91077 Dormitz (DE); Helmecke, Sven, 90427 Nürnberg (DE)
(74) Vertreter: Siemens Healthineers Patent Attorneys

(56) Entgegenhaltungen:
- US-A1- 2003 202 428
- US-A1- 2013 127 467
- US-A1- 2015 271 904
- US-A1- 2016 069 975
- US-A1- 2016 161 581

## Beschreibung

Die Erfindung betrifft einen Magnetresonanztomographen sowie ein Verfahren zum energiesparenden Betrieb des Magnetresonanztomographen. Der Magnetresonanztomograph erkennt eine Zustandsänderung und schlägt ein Energiesparszenario vor.

Magnetresonanztomographen sind bildgebende Vorrichtungen, die zur Abbildung eines Untersuchungsobjektes Kernspins des Untersuchungsobjektes mit einem starken äußeren Magnetfeld ausrichten und durch ein magnetisches Wechselfeld zur Präzession um diese Ausrichtung anregen. Die Präzession bzw. Rückkehr der Spins aus diesem angeregten in einen Zustand mit geringerer Energie wiederum erzeugt als Antwort ein magnetisches Wechselfeld, das über Antennen empfangen wird.

Mit Hilfe von magnetischen Gradientenfeldern wird den Signalen eine Ortskodierung aufgeprägt, die nachfolgend eine Zuordnung von dem empfangenen Signal zu einem Volumenelement ermöglicht. Das empfangene Signal wird dann ausgewertet und eine dreidimensionale bildgebende Darstellung des Untersuchungsobjektes bereitgestellt. Zum Empfang des Signals werden vorzugsweise lokale Empfangsantennen, sogenannte Lokalspulen verwendet, die zur Erzielung eines besseren Signal-Rauschabstandes unmittelbar am Untersuchungsobjekt angeordnet werden.

Die Energieaufnahme eines Magnetresonanztomographen trägt erheblich zu den Betriebskosten bei. Einige Teilsysteme des Magnetresonanztomographen lassen sich schnell deaktivieren und wieder aktivieren. Andere wie die Kühlung oder Stromversorgung hingegen benötigen lange Zeit, um herunterzufahren oder wieder aktiviert zu werden. Dem Magnetresonanztomographen ist aber meist nicht bekannt, wann wieder ein Betrieb aufgenommen werden soll. Aus Gründen der Verfügbarkeit werden deshalb nur jeweils Teilsysteme mit kurzer Anlaufzeit deaktiviert, was aber den Energiespareffekt begrenzt.

Es ist daher eine Aufgabe der Erfindung einen Magnetresonanztomographen und ein Verfahren zum Betrieb bereitzustellen, das höhere Einsparungen erlaubt.

Diese Aufgabe wird von einem Verfahren nach Anspruch 1 sowie einem Magnetresonanztomographen nach Anspruch 8 gelöst.

Das erfindungsgemäße Verfahren ist zum Betrieb eines Magnetresonanztomographen vorgesehen. Der Magnetresonanztomograph weist eine Steuerung auf. Die Steuerung ist zum Steuern einer Bilderfassung vorgesehen. Insbesondere ist die Steuerung aber auch ausgelegt, direkt oder durch Steuerkommandos an untergeordnete Steuerungen, energieintensive Komponenten des Magnetresonanztomographen in einen energiesparenden Ruhezustand versetzen zu können und auch wieder daraus aktivieren zu können. Unter einem energiesparenden Ruhezustand ist insbesondere ein Zustand einer Einheit zu verstehen, bei der der Energieverbrauch der Einheit gegenüber einer Bilderfassung oder einem Bereitschaftsmodus mit einer sehr kurzen Wiederinbetriebnahmezeit kleiner 1 min, 30 3, 10 s oder 1 s bzw. ohne Wiederinbetriebnahmezeit reduziert ist.

In einem Schritt des erfindungsgemäßen Verfahrens erfasst die Steuerung eine Zustandsänderung des Magnetresonanztomographen. Dies kann durch Sensoren erfolgen, die beispielsweise eine Position der Patientenliege oder eines Patienten erfassen. Solange die Patientenliege außerhalb des Patiententunnels ist oder sich kein Patient auf der Patientenliege befindet, ist beispielsweise keine Bilderfassung zu erwarten. Das Erfassen der Zustandsänderung kann auch anhand der Auswertung von Nutzereingaben erfolgen. Wird beispielsweise eine neue Sequenz gestartet, kann für die Dauer der Sequenz keine Energiesparmaßnahme ausgeführt bzw. kein Energiesparszenario umgesetzt werden. Umgekehrt werden auch mit einer internen Zustandsänderung, z.B. mit Beendigung einer Sequenz durch die Steuerung oder das Ablaufen eines Timers seit der letzten Interaktion des Nutzers, wieder das Ausführen von Energiesparszenarien durch Energiesparmaßnahmen möglich.

Insbesondere bewertet die Steuerung die Zustandsänderung, ob diese eine Betriebsunterbrechung indiziert. Mit anderen Worten prüft die Steuerung, ob die Zustandsänderung eine Betriebsunterbrechung, insbesondere mit einer potentiellen Energiesparmaßnahme, ermöglicht, wahrscheinlich macht oder sogar erzwingt. Dies kann beispielsweise geschehen, indem die Steuerung die Zustandsänderung mit einer gespeicherten Datenbank vergleicht. Dort kann beispielsweise zu jeder Zustandsänderung hinterlegt sein, ob diese zu einer Betriebsunterbrechung führt, die eine Energiesparmaßnahme ermöglicht, oder ob häufig im Betrieb darauf eine derartige Betriebsunterbrechung erfolgt. Beispielsweise können Wahrscheinlichkeiten für das Eintreten, durchschnittliche Dauer der Unterbrechung und/oder potenziell zur Energieeinsparung stillegbare Teilsysteme hinterlegt sein. Die Informationen können auch von der Steuerung selbst aus der Beobachtung des laufenden Betriebs gewonnen werden und angepasst werden. Denkbar ist auch, dass aus dem Betrieb vieler Teilsysteme gesammelte Daten ausgewertet und vorab in der Datenbank gespeichert werden.

Die Auswertung kann manuell, maschinell oder insbesondere durch eine künstliche Intelligenz erfolgen. Erfolgt die Auswertung durch eine künstliche Intelligenz in der Steuerung des Magnetresonanztomographen oder einer damit verbundene Steuerung, beispielsweise in einer Cloud, so ist diese in der Lage, die Bewertung an die jeweiligen lokalen Nutzungsgewohnheiten und Nutzerprofil kontinuierlich anzupassen.

In einem weiteren Schritt des erfindungsgemäßen Verfahrens ermittelt die Steuerung mehrere mögliche Energiesparszenarien für eine bevorstehende bzw. potenziell bevorstehende Unterbrechung der Bilderfassung in Abhängigkeit von der Zustandsänderung. Vorzugsweise erfolgt das Ermitteln in Abhängigkeit von dem durch die Zustandsänderung eingetretenen Zustand und/oder einer Dauer einer zu erwartenden Unterbrechung. Beispielsweise sind einige der Teilsysteme in einigen Zuständen nicht erforderlich.

In einem anderen Schritt des erfindungsgemäßen Verfahrens ermittelt die Steuerung Wiederinbetriebnahmezeiten für die ermittelten möglichen Energiesparszenarien. Beispielsweise können für einzelne Teilsysteme in einem Speicher der Steuerung oder in einer Cloud Werte für die Wiederinbetriebnahmeszenarien hinterlegt sein. Sind die betroffenen Teilsysteme unabhängig, so ist für ein Energiesparszenario die längste Wiederinbetriebnahmezeit eines einzelnen betroffenen Teilsystems die Wiederinbetriebnahmezeit für das gesamte Energiesparszenario. Bei wechselseitigen Abhängigkeiten der Teilsysteme ist es auch denkbar, dass sich einzelne Wiederinbetriebnahmezeiten zu einer gesamten Wiederinbetriebnahmezeit addieren.

In einem weiteren Schritt des erfindungsgemäßen Verfahrens wird an einen Nutzer eine Auswahlmöglichkeit für eine Mehrzahl der Energiesparszenarien ausgegeben. Die Auswahlmöglichkeit umfasst mindestens zwei unterschiedliche Energiesparszenarien, die beispielsweise in einer Liste oder Tabelle zur Auswahl angeboten werden. Dies kann beispielsweise auf einem Display an dem Magnetresonanztomograph erfolgen. Insbesondere bietet die Auswahlmöglichkeit auch eine Eingabemöglichkeit für den Nutzer, um eine Auswahl zu tätigen. Dies kann ein Touchscreen sein, Tasten, die an einem Display zugeordnet sind oder auch einfach eine Tastatur oder Maus.

Auf vorteilhafte Weise erlaubt es das erfindungsgemäße Verfahren einem Nutzer mittels seiner Außensicht für die Abläufe um den Magnetresonanztomographen und die interne von dem Verfahren gelieferte Sicht auf die internen Vorgänge des Magnetresonanztomographen einfach einen besonders energiesparenden Betrieb bereitzustellen.

Der erfindungsgemäße Magnetresonanztomograph teilt die Vorteile des erfindungsgemäßen Verfahrens, das auf ihm ausgeführt wird.

Weitere vorteilhafte Ausführungsformen sind zu den Unteransprüchen angegeben.

In einer möglichen Ausführungsform des erfindungsgemäßen Verfahrens weist das Verfahren weiterhin den Schritt des Erfassens einer Auswahl eines Energiesparszenario des Nutzers durch die Steuerung auf. Vorzugsweise erfolgt das Erfassen der Nutzereingabe an einem Gerät, an dem auch die Ausgabe der Auswahlmöglichkeit erfolgt. Dies kann bei einem Display an dem Magnetresonanztomographen ein Eingabeelement wie Taster oder Drehwähler bei dem Display sein oder auch das Display selbst, wenn es als Touchscreen ausgelegt ist. Bei einem abgesetzten Bedienterminal im Nebenraum kann es auch ein Eingabegerät wie eine Tastatur, Maus oder Touchscreen sein. Denkbar wäre auch ein Tablet zur Ausgabe und/oder erfassen der Eingabe. Selbst ein sprachgesteuertes Interface wäre denkbar, bei dem die Auswahlmöglichkeiten akustisch ausgegeben werden und/oder die Auswahl des Nutzers mittels Sprachsteuerung erfasst wird. Auch eine Gestenerkennung mittels Kamera wäre denkbar.

Das durch die Auswahl des Nutzers ausgewählte Energiesparszenario wird anschließend von der Steuerung umgesetzt. Vorzugsweise werden dabei die Teilsysteme entsprechend dem ausgewählten Energiesparszenario in einen energiesparenden Ruhezustand versetzt. Beispielsweise fährt die Steuerung die betroffenen Teilsysteme herunter und unterbricht anschließend die Energieversorgung der entsprechenden Teilsysteme. Bei einfachen Teilsystemen wie z.B. einer Verstimmeinrichtung der Lokalspulen kann es auch ausreichen, direkt die Energieversorgung zu unterbrechen. Denkbar ist auch, dass die Teilsysteme eigene Steuerungen aufweisen und die Steuerung des Magnetresonanztomographen diese durch ein Steuerkommando an die Steuerungen der Teilsysteme in einen Ruhezustand versetzt.

Auf vorteilhafte Weise vereinfacht die Auswahl eines Energiesparszenarios dem Nutzer die Reduktion des Energieverbrauchs.

In einer denkbaren Ausführungsform des erfindungsgemäßen Verfahrens wählt die Steuerung nach einer vorbestimmten Zeit ohne Nutzereingabe nach dem Schritt des Ausgebens das Energiesparszenario mit der kürzesten Wiederinbetriebnahmezeit aus. Anschließend setzt die Steuerung wie bereits beschrieben das Energiesparszenario um.

Auf vorteilhafte Weise ermöglicht die Aktivierung eines Default-Energiesparszenarios auch ohne Nutzereingriff eine Reduzierung des Energieverbrauchs.

In einer möglichen Ausführungsform des erfindungsgemäßen Verfahrens erfolgt in dem Schritt des Ermittelns der Energiesparszenarien das Ermitteln in Abhängigkeit von einem Parameter. Denkbar sind auch mehrere Parameter. Der Parameter kann durch den Magnetresonanztomographen vordefiniert sein, wie beispielsweise eine Zeit zur Wiederinbetriebnahme aus dem Energiesparszenario. Denkbar ist auch die Höhe der zu erzielenden Energieeinsparung. Der Parameter kann auch von der erfassten Zustandsänderung des Magnetresonanztomographen abhängen. Denkbar sind auch Parameter, die von der Nutzung des Magnetresonanztomographen abhängen. Beispielsweise kann eine Zeit durch betriebliche Abläufe der radiologischen Abteilung vorbestimmt sein, wie z.B. eine durch Patientenwechsel mindestens zwischen zwei Bilderfassungen benötigte Zeit.

In einem weiteren Schritt verändert der Nutzer diesen Parameter über einen Nutzerdialog bzw. ist die Steuerung ausgelegt, den Parameter in einem Nutzerdialog zu ändern.

Auf vorteilhafte Weise ist der Nutzer so in der Lage, die Energiespareinstellungen an die betrieblichen Abläufe anzupassen.

Es ist aber ebenso denkbar, dass anstelle der Nutzereingaben ein Algorithmus bzw. künstliche Intelligenz in der Steuerung durch Erfassen der betrieblichen Abläufe vieler Bilderfassungen selbsttätig an die betriebliche Praxis anpasst.

In einer denkbaren Ausführungsform des erfindungsgemäßen Verfahrens sendet die Steuerung in dem Schritt des Ausgebens die Auswahlmöglichkeiten zur Ausgabe an ein abgesetztes Bedieninterface. Dies kann beispielsweise ein abgesetzter Bedienrechner im Nebenraum oder auch ein über ein Datennetz angebundenes Bedienterminal sein. Auch ein drahtlos angebundener Tablett-Computer ist denkbar. Entsprechend empfängt die Steuerung in dem Schritt der Auswahl eines Energiesparszenarios eine Auswahl des Nutzers von dem Bedieninterface, beispielsweise durch eine Tastatureingabe, Auswahl mit Maus oder Touchscreen.

Auf vorteilhafte Weise ermöglicht ein abgesetztes Bedieninterface auch das Aktivieren einer Energieeinsparung aus der Ferne.

In einer möglichen Ausführungsform des erfindungsgemäßen Magnetresonanztomographen weist der Magnetresonanztomograph Bedienelemente zur Eingabe der Auswahl des Nutzers auf. Dies kann ein Touchscreen direkt am Gerät oder vorzugsweise ein haptisches oder taktiles Bedienelement wie z.B. eine oder mehrere Tasten an dem Magnetresonanztomographen sein. Der Magnetresonanztomograph erfasst die Eingabe des Nutzers zur Auswahl des Energiesparszenarios mittels der Bedienelemente.

Auf vorteilhafte Weise erlaubt das Bedienelement am Magnetresonanztomographen eine unmittelbare und sichere Auswahl.

In einer denkbaren Ausführungsform des erfindungsgemäßen Verfahrens beendet der Magnetresonanztomograph auf eine Nutzereingabe hin in einem Schritt die Energiesparmaßnahme bzw. Energiesparmaßnahmen, die gemäß dem ausgewählten und ausgeführten Energiesparszenario aktiv sind und nimmt die betroffenen Teilsysteme wieder in Betrieb bz. fährt sie hoch. Die Nutzereingabe kann dabei vorzugsweise über ein, mehrere oder alle Bedienelemente des Magnetresonanztomographen erfolgen. Denkbar ist ein dediziertes Bedienelement wie ein Taster zum Aufwecken. Möglich ist als Nutzereingabe zum Beenden auch eine Betätigung von Bedienelementen am Magnetresonanztomographen wie beispielsweise die Steuertasten für die Patientenliege.

Auf vorteilhafte Weise vermeidet das Aufwecken über ein Bedienelement eine Wiederinbetriebnahme durch eine aufwändige Prozedur aufeinanderfolgender Kommandos über das Bedieninterface.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden.

Es zeigen:
- Fig. 1: eine schematische Darstellung eines erfindungsgemäßen Magnetresonanztomographen;
- Fig. 2: eine schematische Darstellung der an einer Ausführungsform des erfindungsgemäßen Verfahrens beteiligten Einheiten des erfindungsgemäßen Magnetresonanztomographen;
- Fig. 3: einen schematischen Ablaufplan einer Ausführungsform des erfindungsgemäßen Verfahrens.

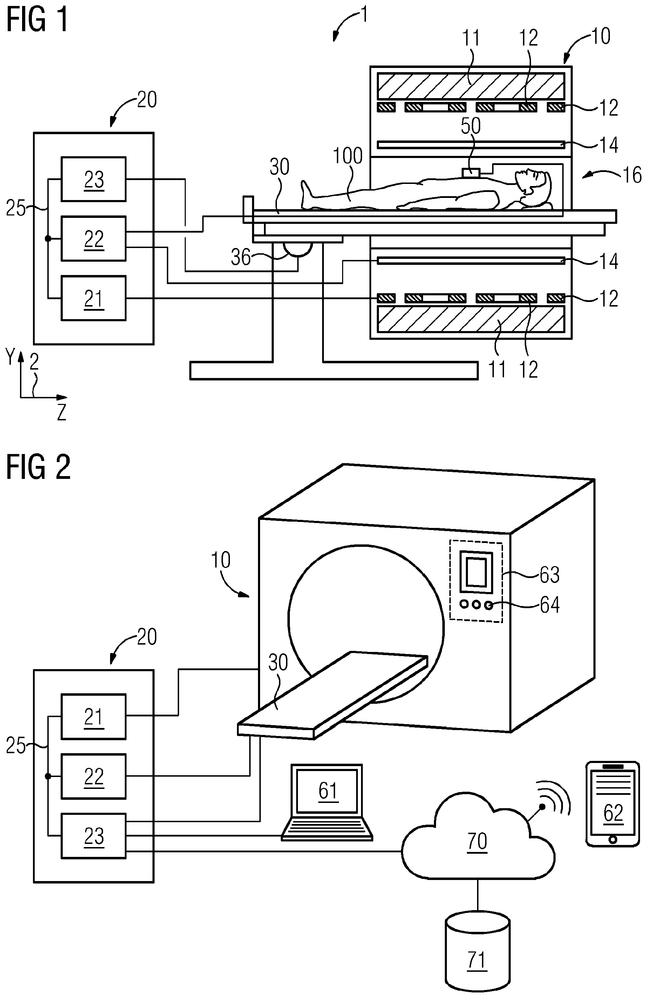

Fig. 1 zeigt eine schematische Darstellung einer beispielhaften Ausführungsform eines erfindungsgemäßen Magnetresonanztomographen 1.

Die Magneteinheit 10 weist einen Feldmagneten 11 auf, der ein statisches Magnetfeld B0 zur Ausrichtung von Kernspins von Proben bzw. Patienten 100 in einem Aufnahmebereich erzeugt. Der Aufnahmebereich ist in einem Patiententunnel 16 angeordnet, der sich in einer Längsrichtung 2 durch die Magneteinheit 10 erstreckt. Ein Patient 100 ist mittels der Patientenliege 30 und der Verfahreinheit 36 der Patientenliege 30 in den Aufnahmebereich verfahrbar. Üblicherweise handelt es sich bei dem Feldmagneten 11 um einen supraleitenden Magneten, der magnetische Felder mit einer magnetischen Flussdichte von bis zu 3T, bei neuesten Geräten sogar darüber, bereitstellen kann. Für geringere Feldstärken können jedoch auch Permanentmagnete oder Elektromagnete mit normalleitenden Spulen Verwendung finden.

Weiterhin weist die Magneteinheit 10 Gradientenspulen 12 auf, die dazu ausgelegt sind, zur räumlichen Differenzierung der erfassten Abbildungsbereiche in dem Untersuchungsvolumen dem Magnetfeld B0 variable Magnetfelder in drei Raumrichtungen zu überlagern. Die Gradientenspulen 12 sind üblicherweise Spulen aus normalleitenden Drähten, die zueinander orthogonale Felder in dem Untersuchungsvolumen erzeugen können.

Die Magneteinheit 10 weist ebenfalls eine Körperspule 14 auf, die dazu ausgelegt ist, ein über eine Signalleitung 33 zugeführtes Hochfrequenzsignal in das Untersuchungsvolumen abzustrahlen und von dem Patient 100 emittierte Resonanzsignale zu empfangen und über eine Signalleitung abzugeben. Bevorzugter Weise wird aber die Körperspule 14 für das Aussenden des Hochfrequenzsignals und/oder das Empfangen durch Lokalspulen 50 ersetzt, die in dem Patiententunnel 16 nahe am Patient 100 angeordnet sind. Es ist aber auch denkbar, dass die Lokalspule 50 zum Senden und Empfangen ausgelegt ist und deshalb eine Körperspule 14 entfallen kann.

Eine Steuereinheit 20 versorgt die Magneteinheit 10 mit den verschiedenen Signalen für die Gradientenspulen 12 und die Körperspule 14 und wertet die empfangenen Signale aus. Eine Magnetresonanztomographen-Steuerung 23 koordiniert dabei die Untereinheiten.

So weist die Steuereinheit 20 eine Gradientenansteuerung 21 auf, die dazu ausgelegt ist, die Gradientenspulen 12 über Zuleitungen mit variablen Strömen zu versorgen, welche zeitlich koordiniert die erwünschten Gradientenfelder in dem Untersuchungsvolumen bereitstellen.

Weiterhin weist die Steuereinheit 20 eine Hochfrequenzeinheit 22 auf, die ausgelegt ist, einen Hochfrequenz-Puls mit einem vorgegebenen zeitlichen Verlauf, Amplitude und spektraler Leistungsverteilung zur Anregung einer Magnetresonanz der Kernspins in dem Patienten 100 zu erzeugen. Dabei können Pulsleistungen im Bereich von Kilowatt erreicht werden. Die einzelnen Einheiten sind über einen Signalbus 25 untereinander verbunden.

Das von Hochfrequenzeinheit 22 erzeugte Hochfrequenzsignal wird über eine Signalverbindung der Körperspule 14 zugeführt und in den Körper des Patienten 100 ausgesendet, um dort die Kernspins anzuregen. Denkbar ist aber auch ein Aussenden des Hochfrequenzsignals über eine oder mehrere Lokalspulen 50.

Die Lokalspule 50 empfängt dann vorzugsweise ein Magnetresonanzsignal aus dem Körper des Patienten 100, denn aufgrund des geringen Abstandes ist das Signal-zu-Rauschverhältnis (SNR) der Lokalspule 50 besser als bei einem Empfang durch die Körperspule 14. Das von der Lokalspule 50 empfangene MR-Signal wird in der Lokalspule 50 aufbereitet und an die Hochfrequenzeinheit 22 des Magnetresonanztomographen 1 zur Auswertung und Bilderfassung weitergeleitet. Vorzugsweise wird dazu die Signalverbindung 33 genutzt, es ist aber beispielsweise auch eine drahtlose Übertragung denkbar.

Teilsysteme mit hohem Energieverbrauch sind die Magneteinheit 10 mit der Kühlung für den Feldmagneten 11, die Gradientensteuerung 21, die über Verstärker die Gradientenspulen 12 mit Strom versorgt sowie die Hochfrequenzeinheit 22, die die hochfrequenten Anregungspulse zur Anregung der Kernspins erzeugt. Aber auch die Verstimmung der Lokalspulen 50 durch die Hochfrequenzeinheit 22 erfordert jeweils eine nennenswerte Leistung zum Durchschalten von PIN-Dioden.

In der in Fig. 1 dargestellten Ausführungsform erfolgt die Ausführung des erfindungsgemäßen Verfahrens durch die Steuerung 23 der Steuereinheit 20.

Fig. 2 zeigt eine schematische Darstellung der an einer Ausführungsform des erfindungsgemäßen Verfahrens beteiligten Einheiten des erfindungsgemäßen Magnetresonanztomographen 1.

Exemplarisch für die in den Energiesparszenarien einzustellenden Einheiten stehen hier die Magneteinheit 10, die Gradientensteuerung 21 und die Hochfrequenzeinheit 22, die von der Steuerung 23 über einen Bus 25 durch Kommandos gesteuert werden. Die Magneteinheit 10 ist über eine separate Steuerleitung 26 mit der Steuerung 23 verbunden. Denkbar sind aber auch andere Konstellationen, in denen beispielsweise alle diese Einheiten in einem Gehäuse kompakt vereint sind und über den Steuerbus 25 verknüpft sind. Möglich ist aber auch ein verteiltes Magnetresonanzsystem, in dem nur die wegen der zu übertragenden Leistung oder Signale in unmittelbarer Nähe erforderlichen Einheiten wie Gradientensteuerung 21 und Hochfrequenzeinheit 22 mit der Magneteinheit 10 vereint sind und die Steuerung abgesetzt über ein Netzwerk in einer Cloud 70 realisiert sind, beispielsweise auf einem Server 71.

Beispielhaft für Teilsysteme, die Zustandsänderungen des Magnetresonanztomographen 1 verursachen können, steht hier die Patientenliege 30, die ebenfalls von der Steuerung 23 gesteuert wird oder diese, beispielsweise über einen Endschalter, informiert, ob sie in den Patiententunnel 16 ein- oder ausgefahren ist. Denkbar sind auch andere Sensoren, die die Anwesenheit eines Patienten und/oder dessen Position erfassen, wie beispielsweise eine Kamera, Lichtschranke oder Drucksensormatte.

Zustandsänderungen des Magnetresonanztomographen 1 können auch über ein Bedieninterface durch einen Nutzer verursacht werden können. In Fig. 2 sind beispielhaft ein Bedienrechner 61 bzw. Bedienterminal, ein Display 63 mit Bedienelementen 64 oder auch ein drahtloser Tablet-Rechner 62 dargestellt. Dabei ist es denkbar, dass die Steuerung 23 über eines, mehrere oder alle diese Bedieninterfaces Ausgaben an einen Nutzer ausgibt oder Eingaben empfängt. Es ist auch ein Betrieb allein mit einem dieser Bedieninterfaces denkbar.

Es ist auch denkbar, dass dedizierte Bedienelemente 64 beispielsweise an der Magneteinheit 10 vorgesehen sind, um die Auswahl einer Energiesparoption direkt am Gerät zu vereinfachen.

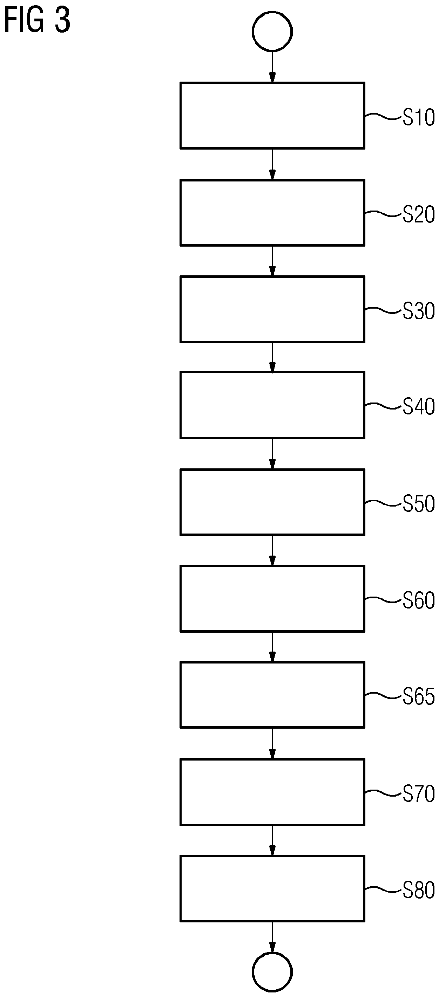

Fig. 3 zeigt einen schematischen Ablaufplan einer Ausführungsform des erfindungsgemäßen Verfahrens. Der erfindungsgemäße Magnetresonanztomograph 1 ist ausgelegt, das Verfahren auszuführen.

In einem Schritt S20 erfasst die Steuerung 23 eine Zustandsänderung des Magnetresonanztomographen 1. Die Zustandsänderung kann durch einen Sensor erfasst werden, wie beispielsweise durch die zuvor beschriebenen Sensoren an der Patientenliege 30. Die Zustandsänderung kann auch über eines der beschriebenen Bedieninterfaces erfasst werden, wenn beispielsweise der Nutzer bzw. Bediener des Magnetresonanztomographen 1 eine Eingabe macht, die einen Zustand des Magnetresonanztomographen 1 verändert. Denkbar ist auch eine systemintern verursachte Zustandsänderung, wie beispielsweise Beendigung eines zuvor ablaufenden Vorgangs, wie beispielsweise eine Bilderfassung oder Teil davon, eines Test- oder Wartungsvorgangs, ein Herauf- oder Herunterfahren von Teilsystemen oder des ganzen Magnetresonanztomographen 1. Die Zustandsänderung wird daraufhin bewertet, ob diese eine Betriebsunterbrechung indiziert, also zumindest mit einer gewissen Wahrscheinlichkeit größer Null für die auf die Zustandsänderung folgende Zeit zu erwarten ist. So schließt beispielsweise der Start einer Sequenz oder eines Tests eine Betriebsunterbrechung für alle Einheiten oder zumindest betroffene Teilsysteme aus. Umgekehrt deuten andere Zustandsänderung wie ein Abbruch einer Aufnahmesequenz, das Herausfahren des Patientenliege 30 oder die Abwesenheit eines Patienten 100 auf der Patientenliege 30 hingegen eine längere Unterbrechung an. Mit Einheiten werden dabei die Teilsysteme des Magnetresonanztomographen 1 bezeichnet, die sich gesteuert von der Steuerung 23 in einen energiesparenden Ruhezustand versetzen lassen, wie die z.B. zur Fig. 2 erläuterte Magneteinheit 10, Gradienteneinheit 21 oder Hochfrequenzeinheit 22, oder auch Subsysteme davon wie Hochfrequenzleistungsverstärker und Verstimmeinheit.

Indiziert die Zustandsänderung eine Betriebsunterbrechung zumindest für eine vorbestimmte minimale Zeit, beispielsweise für mehr als 1 min, mehr als 5 min, mehr als 20 min oder über mehrere Stunden, ermittelt die Steuerung 23 in einem Schritt S30 mehrere mögliche Energiesparszenarien für eine bevorstehende Unterbrechung der Bilderfassung in Abhängigkeit von der Zustandsänderung. Vorzugsweise werden dabei mögliche Energiesparmaßnahmen für Einheiten bzw. Teilsysteme des Magnetresonanztomographen 1 betrachtet, die aufgrund der erfassten Zustandsänderung unmittelbar nicht benötigt werden und deshalb in einen energiesparenden Ruhezustand versetzt werden können. Ein Energiesparszenario umfasst dabei mindestens eine Energiesparmaßnahme für mindestens eine Einheit, die in einen energiesparenden Ruhezustand versetzt werden kann. Vorzugsweise umfasst ein Energiesparszenario aber eine Mehrzahl an Einheiten und/oder Energiesparmaßnahmen, die gemeinsam in dem Zustand des Magnetresonanztomographen 1 nach der Zustandsänderung nicht benötigt werden und gemeinsam in einen energiesparenden Ruhezustand versetzt werden. Beispielsweise können in einem Energiesparszenario mehrere Einheiten zusammengefasst sein, die funktional zusammenhängen oder ähnliche jeweilige Wiederinbetriebnahmezeiten aufweisen. Vorzugsweise umfasst ein Energiesparszenario alle Einheiten, die sich aufgrund des durch die Zustandsänderung eingetretenen Zustandes des Magnetresonanztomographen 1 in einen energiesparenden Ruhezustand versetzen lassen und innerhalb einer für das Energiesparszenario vorbestimmten maximalen Zeit wieder in Betrieb nehmen lassen.

In einem weiteren Schritt S40 ermittelt die Steuerung 23 Wiederinbetriebnahmezeiten für die ermittelten Energiesparszenarien. Bei einem Energiesparszenario mit einer einzelnen betroffenen Einheit ist die Wiederinbetriebnahmezeit für das Energiesparszenario die Wiederinbetriebnahmezeit der einzelnen Einheit. Sind mehrere Einheiten Teil des Energiesparszenarios, so kann die Wiederinbetriebnahmezeit des Energiesparszenarios der Wert der größten Wiederinbetriebnahmezeit einer Einheit des Energiesparszenarios sein. Bei Abhängigkeiten voneinander kann es sich aber auch um eine Summe von Wiederinbetriebnahmezeiten der beteiligten Einheiten handeln. Denkbar sind auch Mischformen. Vorzugsweise ist die Wiederinbetriebnahmezeit für alle möglichen Einheiten für die Energiesparszenarien in einem Speicher der Steuerung 23 oder auf einem Server 71 hinterlegt.

In einem weiteren Schritt S50 gibt die Steuerung 23 eine Auswahlmöglichkeit für eine Mehrzahl der Energiesparszenarien mit einer Information zur jeweiligen Wiederinbetriebnahmezeit an einen Nutzer über ein Bedieninterface aus. Denkbar ist beispielsweise die Ausgabe einer Liste oder Tabelle der Energiesparszenarien auf einem Display 63 einem Bedienterminal 61 oder einem Table-Rechner 62 aus. Möglich wäre aber z.B. auch eine Sprachausgabe.

In einem weiteren Schritt S70 setzt die Steuerung 23 eines der ermittelten Energiesparszenarien um. Welches, hängt dabei von der Reaktion des Nutzers ab, wie nachfolgend zu den Unteransprüchen näher erläutert wird. Das Umsetzen erfolgt dabei dadurch, dass die Steuerung 23 die entsprechenden Einstellbefehle an die betroffenen Einheiten bzw. deren Steuerungen zur Herstellung der Ruhezustände ausgibt oder bei einfachen Einheiten auch direkt die Energiezufuhr unterbricht.

In einer bevorzugten Ausführungsform des erfindungsgemäßen Verfahrens weist das Verfahren den Schritt S60 auf, eine Auswahl eines Energiesparszenarios durch den Nutzer mit der Steuerung 23 zu erfassen. Die Auswahl erfolgt vorzugsweise an einem Bedieninterface wie den beispielhaft dargestellten Bedienrechner 61, dem Display 63 in Verbindung mit dem bzw. den Bedienelementen 64 oder auch einem Tablet-Rechner 62. Es wird beispielsweise eine Liste oder Tabelle der ermittelten Energiesparszenarien und der jeweiligen Wiederinbetriebnahmezeiten dargestellt, z.B. in Zahlen, Symbole oder durch Farbcodierung, und die Auswahl erfolgt durch Eingabe an einer Tastatur, Maus oder Touch Screen. Die Bedienelemente 64 sind den Energiesparszenarien auf dem Display zugeordnet, beispielsweise durch Position, haptische oder visuelle Markierung. Denkbar wäre auch eine akustische Ausgabe und Auswahl durch eine Spracheingabe oder Gesten, die durch eine Kamera erfasst werden.

Die Umsetzung der Auswahl erfolgt anschließen wie bereits beschrieben durch die Steuerung 23.

In einer möglichen Ausführungsform des erfindungsgemäßen Verfahrens interpretiert die Steuerung 23 nach einer vorbestimmten Zeit ohne Nutzereingabe nach dem Schritt S50 des Ausgebens dies ebenfalls als Auswahl des Nutzers und nimmt einen vorbestimmten Default-Wert als Eingabe des Nutzers an. Die vorbestimmte Zeit kann beispielsweise kleiner als 1 min, 5 min, 10 min oder 1 Stunde sein. Vorzugsweise wählt die Steuerung als vorbestimmten Default-Wert dabei das ermittelte Energiesparszenario mit der kürzesten Wiederinbetriebnahmezeit an. Denkbar wäre aber beispielsweise auch ein durch den Nutzer wie nachfolgend beschrieben konfigurierbares Default-Energiesparszenario.

In einer denkbaren Ausführungsform des erfindungsgemäßen Verfahrens erfolgt in dem Schritt S30 das Ermitteln der Energiesparszenarien in Abhängigkeit von einem Parameter. Denkbar ist es beispielsweise, dass der Nutzer das bereits beschriebene Default-Energiesparszenario als einen Parameter angeben kann. Es ist auch möglich, dass bestimmten Zustandsänderungen zu erwartende Betriebsunterbrechungen zugeordnet werden können. Denkbar wäre auch eine einstellbare Zeitabhängigkeit, sodass sich beispielsweise die Parameter zu bestimmten Tageszeiten ändern.

Das Verändern des oder der Parameter erfolgt dabei in einem Schritt S10 als Dialog über ein Bedieninterface mit dem Nutzer. Der bzw. Die Parameter werden dann von der Steuerung 23 entsprechend der Eingabe des Nutzers geändert.

In einer möglichen Ausführungsform des erfindungsgemäßen Verfahrens gibt die Steuerung in Schritt S50 die Auswahlmöglichkeiten zur Ausgabe an ein abgesetztes Bedieninterface aus, beispielsweise einen Bedienrechner 61 oder Tablet-Rechner 62, die über ein drahtgebundenes oder drahtloses Datennetz mit der Steuerung 23 verbunden sind. Entsprechend empfängt die Steuerung in Schritt S60 auf umgekehrtem Weg die Auswahl des Nutzers von dem Bedieninterface. Auf vorteilhafte Weise ist ein tieferer und effektiverer Energiesparzustand mit längerer Wiederinbetriebnahmezeit gerade dann sinnvoll, wenn kein Nutzer am Gerät ist. Die Energiesparmaßnahmen können so beispielsweise über eine zentrales und immer besetztes Service-Zentrum optimiert werden. Denkbar wäre auch eine zentrale Steuerung durch einen Server oder künstlichen Intelligenz als virtueller Nutzer im Service-Zentrum.

In einer denkbaren Ausführungsform des erfindungsgemäßen Verfahrens beendet die Steuerung 23 in einem Schritt S80 auf eine Nutzereingabe hin die Energiesparmaßnahme bzw. das Energiesparszenario und nimmt die Einheiten, die gemäß dem Energiesparszenario in einen energiesparenden Ruhezustand versetzt wurden, wieder in Betrieb.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen, welcher durch die Ansprüche gegeben ist.

## Patentansprüche

1. Verfahren zum Betrieb eines Magnetresonanztomographen (1) mit einer Steuerung (23) zum Steuern einer Bilderfassung, wobei das Verfahren die Schritte aufweist:
Erfassen (S20) einer Zustandsänderung des Magnetresonanztomographen (1) durch die Steuerung (23) und Bewerten der Zustandsänderung durch die Steuerung (23), ob diese eine Betriebsunterbrechung indiziert;
Ermitteln (S30) mehrerer möglicher Energiesparszenarien durch die Steuerung (23) für eine bevorstehende Unterbrechung der Bilderfassung in Abhängigkeit von der Zustandsänderung;
Ermitteln (S40) von Wiederinbetriebnahmezeiten für die möglichen Energiesparszenarien durch die Steuerung (23);
Ausgeben (S50) einer Auswahlmöglichkeit für eine Mehrzahl der Energiesparszenarien mit einer Information zur jeweiligen Wiederinbetriebnahmezeit durch die Steuerung (23) an einen Nutzer;
Umsetzen (S70) eines Energiesparszenarios durch die Steuerung (23).

2. Verfahren nach Anspruch 1, wobei das Verfahren weiterhin den Schritt des Erfassens (S60) einer Auswahl eines Energiesparszenario des Nutzers durch die Steuerung (23) aufweist, wobei die Steuerung (23) das ausgewählte Energiesparszenario in Schritt (S70) umsetzt.

3. Verfahren nach Anspruch 1, wobei nach einer vorbestimmten Zeit ohne Nutzereingabe nach dem Schritt (S50) des Ausgebens die Steuerung (23) in Schritt (S65) das Energiesparszenario mit der kürzesten Wiederinbetriebnahme auswählt und anschließend in Schritt (S70) umsetzt.

4. Verfahren nach einem der vorhergehenden Ansprüche, wobei in dem Schritt (S30) des Ermittelns der Energiesparszenarien das Ermitteln in Abhängigkeit von einem Parameter erfolgt, wobei das Verfahren weiterhin in einem Schritt (S10) verändern des Parameters die Steuerung (23) in einem Nutzerdialog in Abhängigkeit von einer Nutzereingabe den Parameter ändert.

5. Verfahren nach einem der Ansprüche 2 bis 4, wobei die Steuerung (23) in Schritt (S50) die Auswahlmöglichkeiten zur Ausgabe an ein abgesetztes Bedieninterface sendet und die Auswahl des Nutzers in Schritt (S60) von dem Bedieninterface empfängt.

6. Verfahren nach Anspruch 2, wobei der Magnetresonanztomograph (1) Bedienelemente zur Eingabe der Auswahl des Nutzers aufweist und mit diesen in Schritt (S60) die Auswahl erfasst.

7. Verfahren nach einem der vorhergehenden Ansprüche, wobei der Magnetresonanztomograph (1) auf eine Nutzereingabe in einem Schritt (S80) das Energiesparszenario beendet.

8. Magnetresonanztomograph mit einer Steuerung (23), wobei die Steuerung (23) ausgelegt ist, das Verfahren nach einem der Ansprüche 1 bis 7 auszuführen.

9. Computerprogrammprodukt, welches in eine Speichereinheit einer Steuerung (23) eines Magnetresonanztomographen (1) nach Anspruch 8 ladbar ist, aufweisend Programmcode-Mittel, um ein Verfahren nach einem der Ansprüche 1 bis 7 auszuführen, wenn das Computerprogrammprodukt in der Steuerung (23) des Magnetresonanztomographen (1) nach Anspruch 8 ausgeführt wird.

10. Computer-lesbares Medium, auf dem Programmcode-Mittel gespeichert sind, die von einer Steuerung (23) eines Magnetresonanztomographen (1) nach Anspruch 8 gelesen werden können und von der Steuerung (23) ausgeführt werden können, um ein Verfahren nach einem der Ansprüche 1 bis 7 auszuführen.

## Claims

1. Method for operating a magnetic resonance tomography system (1) having a control system (23) for controlling an image recording, wherein the method has steps as follows:
capturing (S20), by means of the control system (23), a change in state of the magnetic resonance tomography system (1) and
assessing, by means of the control system (23), whether the change in state indicates an operating interruption;
determining (S30), by means of the control system (23), a plurality of possible energy-saving scenarios for an imminent interruption of the image recording as a function of the change in state;
determining (S40), by means of the control system (23),
restart times for the possible energy-saving scenarios;
outputting (S50), by means of the control system (23), a selection possibility for a plurality of energy-saving scenarios, with information relating to the respective restart times, to a user;
implementing (S70), by means of the control system (23), an energy-saving scenario.

2. Method according to claim 1, wherein the method further comprises the step of capturing (S60) by means of the control system (23) a user selection of an energy-saving scenario, wherein the control system (23) implements the selected energy-saving scenario in step (S70).

3. Method according to claim 1, wherein after a predetermined time without user input following the output in step (S50), the control system (23) selects the energy-saving scenario having the shortest restart time in step (S65) and then implements this energy-saving scenario in step (S70).

4. Method according to one of the preceding claims, wherein in the step (S30) for determining the energy-saving scenarios, the determination takes place as a function of a parameter, wherein in a step (S10) for varying the parameter, the control system (23) changes the parameter as a function of a user input in a user dialog.

5. Method according to one of claims 2 to 4, wherein the control system (23) sends the selection possibilities to a remote operating interface for output in step (S50) and receives the user selection from the operating interface in step (S60).

6. Method according to claim 2, wherein the magnetic resonance tomography system (1) has operating elements for inputting the user selection and captures the selection via these operating elements in step (S60).

7. Method according to one of the preceding claims, wherein the magnetic resonance tomography system (1) terminates the energy-saving scenario in response to a user input in step (S80).

8. Magnetic resonance tomography system having a control system (23), wherein the control system (23) is designed to execute the method according to one of claims 1 to 7.

9. Computer program product which can be loaded into a memory unit of a control system (23) of a magnetic resonance tomography system (1) according to claim 8, having program code means for executing a method according to one of claims 1 to 7 when the computer program product is executed in the control system (23) of the magnetic resonance tomography system (1) according to claim 8.

10. Computer-readable medium on which are stored program code means that can be read by a control system (23) of a magnetic resonance tomography system (1) according to claim 8 and can be executed by the control system (23) in order to execute a method according to one of claims 1 to 7.

## Revendications

1. Procédé pour faire fonctionner un tomodensitomètre (1) à résonance magnétique, comprenant une commande (23) pour la commande d'une prise d'images, dans lequel le procédé comporte les stades :
détection (S20) d'une modification d'état du tomodensitomètre (1) à résonance magnétique par la commande (23) et évaluation de la modification d'état par la commande (23) sur le point de savoir, si celle-ci indique une interruption du fonctionnement ;
détermination (S30), en fonction de la modification d'état, de plusieurs scénarii d'économie d'énergie possibles par la commande (23) pour une interruption imminente de la prise d'images ;
détermination (S40) de temps de remise en fonctionnement pour les scénarii d'économie d'énergie possibles par la commande (23) ;
envoi (S50) à un utilisateur d'une possibilité de sélection pour une pluralité des scénarii d'économie d'énergie par une information pour le temps respective de remise en fonctionnement par la commande (23);
changement (S70) d'un scénario d'économie d'énergie par la commande (23).

2. Procédé suivant la revendication 1, dans lequel le procédé comporte en outre le stade de la détection (S60) par la commande (23) d'une sélection d'un scénario d'économie d'énergie de l'utilisateur, dans lequel la commande (23) change de scénario d'économie d'énergie sélectionné dans le stade (S70).

3. Procédé suivant la revendication 1, dans lequel, après un temps déterminé à l'avance sans demande de l'utilisateur après le stade (S50) d'envoi, la commande (23) au stade (S65) sélectionne le scénario d'économie d'énergie ayant la remise en fonctionnement la plus courte et effectue ensuite le changement dans le stade (S70).

4. Procédé suivant l'une des revendications précédentes, dans lequel dans le stade (S30) la détermination des scénarii d'économie d'énergie effectue la détermination en fonction d'un paramètre, dans lequel en outre le procédé, dans un stade (S10) de modification du paramètre, change la commande (23) dans un dialogue avec un utilisateur en fonction d'une demande de l'utilisateur.

5. Procédé suivant l'une des revendications 2 à 4, dans lequel la commande (23) envoie au stade (S50) les possibilités de sélection pour l'envoi à une interface de service à distance et reçoit de l'interface de service au stade (S60) la sélection de l'utilisateur.

6. Procédé suivant la revendication 2, dans lequel le tomodensitomètre (1) à résonance magnétique a des éléments de service pour la demande de la sélection de l'utilisateur et effectue avec ceux-ci la sélection au stade (S60).

7. Procédé suivant l'une des revendications précédentes, dans lequel le tomodensitomètre (1) à résonance magnétique met fin au scénario d'économie d'énergie dans un stade (S80) sur une demande de l'utilisateur.

8. Tomodensitomètre à résonance magnétique comprenant une commande (23), dans lequel la commande (23) est conçue, pour exécuter le procédé suivant l'une des revendications 1 à 7.

9. Produit de programme d'ordinateur, qui peut être chargé dans une unité de mémoire d'une commande (23) d'un tomodensitomètre (1) à résonance magnétique suivant la revendication 8, comportant des moyens de code de programme pour exécuter un procédé suivant l'une des revendications 1 à 7, lorsque le produit de programme d'ordinateur est exécuté dans la commande (23) du tomodensitomètre (1) à résonance magnétique suivant la revendication 8.

10. Support déchiffrable par ordinateur, sur lequel sont mises en mémoire des moyens de code de programme, qui peuvent être déchiffrés par une commande (23) d'un tomodensitomètre (1) à résonance magnétique suivant la revendication 8 et être exécuté par la commande (23), afin d'exécuter un procédé suivant l'une des revendications 1 à 7.
